Europäisches Patentamt

European Patent Office

Office européen des brevets

(11) Veröffentlichungsnummer: **0 480 179 A1**

## EUROPÄISCHE PATENTANMELDUNG

(21) Anmeldenummer: **91115144.7**

(22) Anmeldetag: **07.09.91**

(51) Int. Cl.5: **F02D 19/08**, G01N 33/28

(30) Priorität: **01.10.90 DE 4031009**

(43) Veröffentlichungstag der Anmeldung:
**15.04.92 Patentblatt 92/16**

(84) Benannte Vertragsstaaten:
**DE FR GB IT**

(71) Anmelder: **PIERBURG GMBH**
**Alfred-Pierburg-Strasse 1**
**W-4040 Neuss 1(DE)**

(72) Erfinder: **Härtel, Günter**
**Am Vogelbusch 16**
**W-4040 Neuss 21(DE)**
Erfinder: **Schürfeld, Armin**
**Wagnerplatz 13**
**W-4005 Meerbuch 2(DE)**
Erfinder: **Lösing, Karl-Heinrich, Dr.**
**Heidestrasse 88a**
**W-4234 Alpen(DE)**
Erfinder: **Thönnessen, Dieter**
**Ackerstrasse 29**
**W-4060 Viersen 11(DE)**
Erfinder: **Remde, Ulrich**
**Leipziger Strasse 18**
**W-4005 Meerbusch 3(DE)**

(54) **Verfahren und Einrichtung zur Nutzung von Brennstoffen mit Alkoholzusätzen für eine Brennkraftmaschine.**

(57) Die Erfindung macht sich die Tatsache zunutze, daß heute handelsübliche Brennstoffsorten annähernd gleiche Mol-Gewichte haben, die sich aber deutlich von den Mol-Gewichten von Methanol bzw. Ethanol unterscheiden.

Durch vollständiges Verdampfen eines Brennstoffprobenvolumens mit einer erfindungsgemaßen Einrichtung ergeben sich je nach Anteil der Beimischung von Alkoholen zum normalen Brennstoff unterschiedliche Volumina der verdampften Brennstoffprobe, bzw. bei Begrenzung des Volumens unterschiedliche Drücke, wobei es jeweils notwendig ist, die Probentemperatur zu erfassen und deren Auswirkung auf die Meßgröße zu berücksichtigen.

Aus diesen nach der Verdampfung der Brennstoffprobenmenge gemessenen Werten (Volumen- und/oder Druck- und/oder Temperaturänderung) wird in einem Vergleich mit in einem Steuergerät gespeicherten Bezugswerten ein Korrektursignal gebildet, mit dem Motorsteuerungsgroßen änderbar sind.

Derartige Verfahren und Einrichtungen finden Anwendung bei Brennkraftmaschinen.

**Fig. 1**

Die Erfindung betrifft ein Verfahren und Einrichtung zur Nutzung von Brennstoffen mit Alkoholzusätzen für eine Brennkraftmaschine.

Neben Superbenzin, Normalbenzin und Dieselkraftstoff sind auch Alkohole wie Methanol, Ethanol, Propanol, Isobutanol u. a. als Brennstoffe für Verbrennungsmaschinen geeignet.

Für die Leistung des Motors ist der Heizwert des brennfähigen Brennstoff-Luft-Gemisches maßgebend. Bei stöchiometrischem Mischungsverhältnis liegt der Gemischheizwert für alle praktisch verwendbaren flüssigen Brennstoffe und Flüssiggase zwischen 700 und 735 kcal/kg; sie sind also fast gleich und Leistungseinbußen des Motors durch Zumischen von Methanol u. a., sind nicht zu befürchten. Auch die meisten sonstigen Eigenschaften der Alkohole sind so, daß sie sich ohne weiteres mit üblichen Flüssigbrennstoffen mischen lassen beziehungsweise keine Störungen beim praktischen Motorbetrieb hervorrufen.

Wünschenswert ist eine Alkoholzusammensetzung vor allem aus folgenden Gründen: Alkohole eignen sich als Veredelungsbrennstoff für minderwertige Benzine. Durch Zugabe von zum Beispiel Methanol zu Normalbenzin kann die Klopffestigkeit wesentlich erhöht werden, so daß mit diesem Gemisch auch höher verdichtete Verbrennungsmotoren gefahren werden können. Es werden umweltschädliche Antiklopfmittel entbehrlich. Hinzu kommt, daß Alkohole zunehmend in den USA aus Umweltschutzgründen an Bedeutung gewinnen, insbesondere durch verschärfte Vorschriften in Kalifornien.

Im übrigen lassen sich Alkohole, insbesondere Methanol, auf relativ billige Weise aus Kohle herstellen und sie stehen daher auch in ferner Zukunft in großen Mengen zur Verfügung. Besonders Ethanol ist umweltfreundlich, wenn es aus pflanzlichen Produkten gewonnen wird (biologischer Kreislauf). Alkohole sind daher ein geeignetes Streckmittel für die nur noch begrenzt verfügbaren Erdölbrennstoffe.

Nachteilig ist jedoch, daß der spezifische Mindestluftbedarf für eine vollständige Verbrennung bei Alkoholen geringer ist als bei herkömmlichen Brennstoffen. Bei derselben angesaugten Luftmenge muß dem Motor bei Alkohol- oder Benzin/Alkohol-Mischbetrieb also eine entsprechend höhere Brennstoffmenge zugeführt werden, um ein stöchiometrisches Luft-Brennstoff-Verhältnis zu erreichen. Dies macht eine entsprechende Justierung der Gemischaufbereitungseinrichtung des Verbrennungsmotors erforderlich. Da jedoch gegebenenfalls Alkohole in schwankenden Anteilen zugemischt werden, oder zumindest sich beim Nachtanken unterschiedlicher Brennstoffsorten im Kraftfahrzeugtank ergeben, muß entsprechend dem Alkoholanteil im Brennstofftank das erforderliche Luft-Brennstoff-Verhältnis für stöchiometrisches Gemisch im elektronischen Steuergerät für den Gemischbildner neu bestimmt werden.

In der DE-OS 39 23 992 ist ein Kraftstoffsensor zur Erfassung des Mischungsanteils von Methanol eines Brennstoff-Methanolgemisches offenbart, der zwei im Abstand voneinander angeordnete Elektrodenplatten enthält, die in den Brennstoff eingetaucht sind. Die Dielektrizitätskonstante des Brennstoffes wird durch Messung der Kapazität zwischen den beiden Elektrodenplatten erfaßt, und zwar der zugemischte Anteil an Methanol wird aus der Dielektrizitätskonstanten abgeleitet. Dieses Meßprinzip zeigt jedoch eine starke Anfälligkeit bei Dampfblasenbildung.

Aus der DE-OS 22 59 323 ist eine Vorrichtung zur automatischen Anpassung einer Brennkraftmaschine an die Anforderungen der wahlweisen Verwendung verschiedenartiger flüssiger Brennstoffe bekannt, die einen brennstoffdurchflossenen, bis auf die Zu- und Ablauföffnungen für den Kraftstoff geschlossenen Meßbehälter aufweist, in dem eine Teilmenge des Brennstoffes unter dem Einfluß zugeführter Wärme verdampft wird, wobei die relative Größe des dampfgefüllten Raumes im Inneren des Meßbehälters als Maß zur Anpassung an die verschiedenen Brennstoffarten dient.

Nachteilig hierbei ist, daß nur festgestellt werden kann, ob ein bestimmter Anteil über- oder unterschritten wird.

Hiervon ausgehend liegt der Erfindung die Aufgabe zugrunde, ein Verfahren und eine Einrichtung zur Nutzung von Brennstoffen mit Alkoholzusätzen für eine Brennkraftmaschine eingangs genannter Art derart zu gestalten, daß o.g. Nachteile vermieden werden.

Diese Aufgabe wird durch die Merkmale der Ansprüche 1 bis 8 hinsichtlich der Verfahren gelöst. Die Ansprüche 9 bis 14 beziehen sich auf eine Einrichtung, die nach den Verfahren betrieben wird, und vorteilhafte Weiterbildungen dieser Einrichtung.

Die Erfindung macht sich die Tatsache zunutze, daß heute handelsübliche Brennstoffsorten annähernd gleiche Mol-Gewichte haben, die sich aber deutlich von den Mol-Gewichten von Methanol und Ethanol unterscheiden. So wiegt 1 Mol des handelsüblichen Brennstoffes ca. 98 gr., 1 Mol Ethanol ca. 46 gr. und 1 Mol Methanol ca. 32 gr.

Durch vollständiges Verdampfen eines bestimmten Probenvolumens ergeben sich, je nach Anteil der Beimischung von Alkoholen zum normalen Brennstoff, unterschiedliche Volumina der verdampften Brennstoffprobe. Bei Begrenzung des Volumens ergeben sich in Abhängigkeit der Alkoholbeimengung unterschiedliche Drücke. Zur genauen Bestimmung des Alkoholanteils ist es jeweils notwendig, die Probentemperatur zu erfassen und deren Auswirkung auf die Meßgröße zu berücksichtigen.

Aus diesen nach der Verdampfung der Brennstoffprobenmenge gemessenen Werten (Volumen- und/oder Druck- und/oder Temperaturänderung) wird in einem Vergleich mit in dem Steuergerät gespeicherten Bezugswerten ein Korrektursignal gebildet, mit dem Motorsteuerungsgrößen änderbar sind.

Zur Durchführung des Verfahrens wird eine erfindungsgemäße Einrichtung vorgeschlagen, mit der eine Brennstoffprobenmenge vollständig verdampft wird und die zur Bestimmung des Alkohols und dessen Anteils notwendigen Druck-, Volumen-, Temperaturänderungen erfaßt werden.

Mit dieser Erfindung wird es möglich, Alkohole und deren prozentuale Anteile im Brennstoff zu erkennen, in dem zum Beispiel nach jedem Motorstart eine Probe verdampft wird, nach deren Ergebnis eine Beeinflussung der Brennkraftmaschine erfolgen kann.

Die erfindungsgemäße Einrichtung und deren Anwendung auf das erfindungsgemäße Verfahren hat den Vorteil, daß eine weitgehende Unempfindlichkeit gegen Qualitätsschwankungen (Sommer/Winter) des Brennstoffes gegeben ist. Ferner liegt Dampfblasenunempfindlichkeit vor.

Anhand der Fig. 1 bis 3 wird die Erfindung näher erläutert. Es zeigt:

Fig. 1 einen Schnitt durch eine Einrichtung zur Verdampfung einer Brennstoffprobenmenge;

Fig. 2 einen Schnitt durch eine andere Ausführungsform einer Einrichtung zur Verdampfung einer Brennstoffprobenmenge;

Fig. 3 einen Schnitt durch eine weitere Ausführungsform einer Einrichtung zur Verdampfung einer Brennstoffprobenmenge.

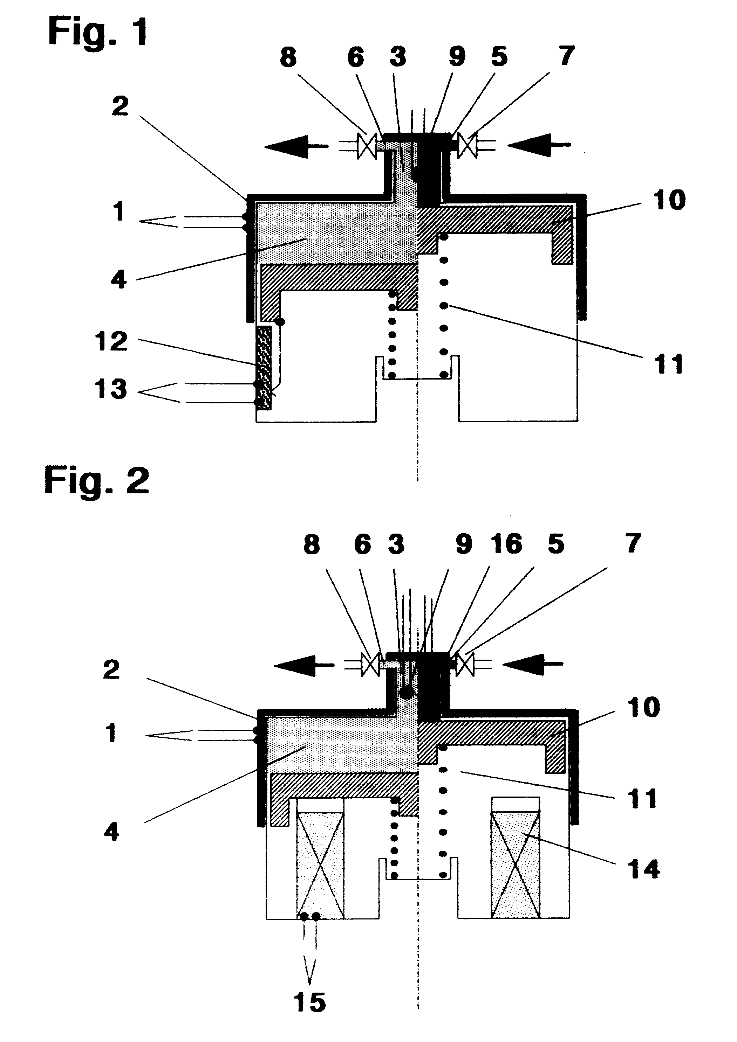

Der rechte Teil der Schnittdarstellung der Fig. 1 bis 3 zeigt die Einrichtung in Ruhestellung, der linke Teil die Einrichtung während eines Meßzyklus.

Fig. 1 zeigt eine Einrichtung zur Verdampfung einer Brennstoffprobenmenge bestehend aus einem über elektrische Anschlüsse 1 beheizbaren Gehäuse 2, das eine Probennahmekammer 3, die sich zu einer Verdampfungskammer 4 aufweitet, umfaßt, wobei die Probennahmekammer 3 Zu- und Ablaufkanäle 5, 6, die mit Hilfe von Ventilen 7, 8 geöffnet und geschlossen werden, und einen Temperatursensor 9 aufweist. In der Verdampfungskammer 4 ist ein Kolben 10 angeordnet, der gegen die Kraft einer Feder 11 axial verschiebbar ist und dessen Position von einem Sensor 12 z. B. einem Potentiometer oder optischen Sensor erfaßt und über elektrische Anschlüsse 13 an ein nicht dargestelltes Steuergerät weitergegeben wird.

**Funktionsweise unter Anwendung des Verfahrens nach Anspruch 1**

Liegt kein Meßzyklus vor (Ruhestellung), strömt Brennstoff kontinuierlich über Ventil 7 und Zulaufkanal 5 in die Probennahmekammer 3 und verläßt diese wieder über den Ablaufkanal 6 und das Ventil 8, wodurch ständig, für den Fall einer Messung, frischer Brennstoff vorliegt. Der Kolben 10 liegt aufgrund der Kraft der Feder 11 am Gehäuseboden an und verschließt den zur Verdampfungskammer 4 hin geöffneten Bereich der Probennahmekammer 3.

Sobald ein Meßzyklus vorliegt, wird der Zulaufkanal 5 über das Ventil 7 geschlossen. Die Brennstoffprobenmenge wird nach Konditionierung auf eine konstante Temperatur (z. B. 30° C) und einen Druck Po und nach Schließen des Ablaufkanals 6 über das Ventil 8 in einem ersten Verfahrensschritt auf eine vorgegebene Temperatur (z. B. 220° C) erhitzt, bis die Brennstoffprobenmenge verdampft ist. In einem zweiten Verfahrensschritt wird die nach der Verdampfung entstandene Volumenänderung, die sich in einer veränderten Position des Kolbens 10 darstellt, durch ein Potentiometer oder einen optischen Sensor festgestellt. Dieser Wert wird in einem dritten Verfahrensschritt unter Berücksichtigung der Temperatur mit dem im Steuergerät gespeicherten Bezugswert für normalen Brennstoff verglichen und aus der Abweichung (Differenz) ein Korrektursignal gebildet, mit dem im vierten Verfahrensschritt Motorsteuerungsgrößen wie z. B. die Brennstoff- bzw. Luftzufuhr änderbar sind.

Das gebildete Korrektursignal wird in einem fünften Verfahrensschritt gespeichert, bis es bei erneuter Brennstoffprobenmessung bestätigt bzw. geändert wird.

Damit ist der Meßzyklus der Verdampfungseinrichtung beendet, so daß in einem sechsten Verfahrensschritt die Brennstoffprobenbeheizung abgeschaltet und der Ablaufkanal 6 geöffnet wird, das Volumen verkleinert sich, der Kolben 10 legt sich am Gehäuseboden an. Der Zulaufkanal 5 wird geöffnet und die Probennahmekammer gespült und gekühlt.

Bei einer anderen Ausführungsform einer Verdampfungseinrichtung nach Fig. 2 ist in der Verdampfungskammer 4 ein Hubmagnet 14 angeordnet, der über elektrische Anschlüsse 15 über das nicht dargestellte Steuergerät ansteuerbar ist. Zusätzlich zu dem Temperatursensor 9 weist die Probennahmekammer 3 einen Drucksensor 16 auf.

Bei dieser Ausführungsform kommt das Verfahren nach Anspruch 2 zum Einsatz, in dem im ersten Verfahrensschritt nach der Konditionierung der Brennstoffprobenmenge und Schließen des Ventils 8 der Probennahmekammer 3 ein vorgegebenes Volumen durch Bestromen des Hubmagneten 14 zugeschaltet wird, d. h. der Kolben 10 wird von dem Hubmagnet 14 angezogen, bis ein definiertes Volumen erreicht ist.

Der Kolben 10 kann auch durch unteratmosphärischen Druck verstellt werden. Dann wird die Brennstoffprobenmenge bei einer kon

stanten Temperatur (z. B. 120°C) verdampft.

Das Verdampfen zieht eine Druckänderung nach sich, dessen Wert im zweiten Verfahrensschritt von dem Drucksensor 16 erfaßt wird, wobei dann im dritten Verfahrensschritt dieser Druckwert unter Berücksichtigung der beiden Konstanten (Temperatur und Volumen) mit im Steuergerät gespeicherten Bezugswerten für normalen Brennstoff verglichen wird und aus der Abweichung ein Korrektursignal gebildet wird.

Der fünfte und sechste Verfahrensschritt laufen ähnlich ab, wie bereits bei dem Verfahren, nach dem die Einrichtung nach Fig. 1 arbeitet, beschrieben. Nach Bildung und Speicherung des Korrekturwertes wird die Beheizung abgeschaltet, die Stromzuführung zum Hubmagneten 14 unterbrochen, der Ablaufkanal 6 über Ventil 8 geöffnet und die Probennahmekammer 3 entspannt, wodurch es zur Rückkondensation des Gases zu Flüssigkeit kommt.

Nach Öffnen des Zulaufkanals 5 durch Ventil 7 sorgt frischer Brennstoff für Spülung und Kühlung der Probennahmekammer 3 und die Verdampfungseinrichtung ist bereit für einen neuen Meßzyklus.

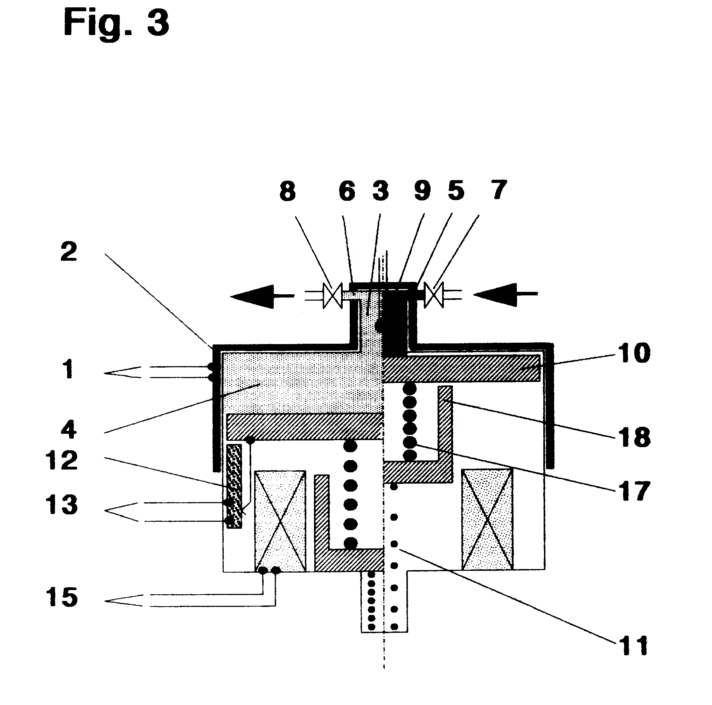

Eine weitere Ausführungsform nach Fig. 3 weist in der Verdampfungskammer 4 einen zweiten mit dem Kolben 10 über eine Zugfeder 17 verbundenen als Hubanker ausgebildeten Kolben 18 auf, der axial verschiebbar gegen die Feder 11 wirkt. Die übrigen Bauteile dieser Einrichtung sind aus der Fig. 1 und 2 und deren Beschreibung bereits bekannt.

Bei dieser Ausführungsform wird nach dem Verfahren nach Anspruch 3 nach der Konditionierung der Brennstoffprobenmenge und Schließen der Probennahmekammer 3 im ersten Verfahrensschritt ein Volumen zugeschaltet und solange vergrößert, bis sich ein vorgegebener Druck im Volumen eingestellt hat. Das geschieht dadurch, daß der Hubmagnet 14 bestromt wird und den Hubanker 18 auf einen am Verdampfungskammerboden befindlichen Anschlag zieht. Über die Zugfeder 17 wird der Kolben 10 in eine von der Zusammensetzung und Temperatur der Brennstoffprobenmenge abhängige Position gezogen, die von dem Potentiometer 12 erfaßt wird.

Während der Erhitzung und dem Verdampfen der Brennstoffprobenmenge bei konstanter Temperatur von z. B. 220°C wandert der Kolben infolge des Ausgleichs des Druckanstieges weiter in Richtung Hubanker 18 und nimmt eine vom Anteil des Alkohols abhängige Position ein.

Diese Position wird nach Verfahrensschritt 2 vom Sensor 12, der auch als berührungsloser Sensor ausgebildet sein kann, gemessen und dient als Basiswert zur Korrektursignalbildung nach Verfahrensschritt 3. Die sich anschließenden Verfahrensschritte 4 bis 6 laufen wie bereits beschrieben ab.

Eine ähnlich der Fig. 3 ausgebildete Ausführungsform einer Einrichtung zur Verdampfung einer Brennstoffprobenmenge wird nach dem Verfahren nach Anspruch 4 betrieben und weist einen Endlagenschalter zur Erkennung der Position des Kolben 10 auf, wobei hier der Kolben 10 zuerst über den Hubmagneten 14 und die Zugfeder 17 in eine definierte Lage (Volumen) gebracht wird und dann die Temperatur so geregelt (z. B. > 220°C) wird, bis der Kolben 10 den Endlagenschalter erreicht. Druck und Volumen sind somit konstant, während der Temperaturwert zur Korrektursignalbildung nach oben beschriebenem Verfahren herangezogen wird.

Mit den beiden letzten Varianten entfällt der Einsatz des recht teuren Drucksensors.

Das von den einzelnen Ausführungsformen aufgrund der Druck- und/oder Volumen- und/oder Temperaturänderung im dritten Verfahrensschritt gebildete und im fünften Verfahrensschritt gespeicherte Korrektursignal kann auch bei einem Wiederstart der Brennkraftmaschine verwendet werden, bis es nach erneuter Brennstoffprobenmessung bestätigt bzw. aktualisiert wird.

Die sich immer weiter verschärfenden Abgasvorschriften machen es erforderlich, daß auch kleine und geringste Arbeitszeiträume einer Brennkraftmaschine (zum Beispiel Kaltstart, Warmlaufphase, Heißstart und so weiter) immer mehr einer Optimierung der Abgasemissionen unterzogen werden müssen. Aus diesem Grund wird das bisher beschriebene Verfahren wie folgt weiter verbessert.

Bei einem Start (Kalt-Heißstart) der Brennkraftmaschine wird sofort ein Meßzyklus zur Korrektursignalbildung ausgelöst, ein neues Korrektursignal steht jedoch erst nach einer gewissen Zeit zur Verfügung, die benötigt wird, um die Verfahrensschritte eins bis fünf zu durchlaufen.

Für den Start und bis zur Vorlage eines aktuellen Korrektursignals wird das letzte gespeicherte Korrektursignal verwendet.

Nach dem Start werden generell mehrere Messungen in Folge durchgeführt, bis sich der Meßwert stabilisiert hat.

Damit wird erreicht, daß nach einem Tankstop die momentan den Einspritzventilen zugeführte Brennstoffmischung erfaßt wird, wenn die Meßeinrichtung vorzugsweise in der Rücklaufleitung zum Tank angeordnet wird.

Es ist jedoch auch denkbar, das letzte gespeicherte Korrektursignal in Abhängigkeit des Füllstandes des Brennstofftanks zu korrigieren beziehungsweise solange zu übersteuern, bis ein neues Korrektursignal der Verdampfungseinrichtung vorliegt.

Hierbei wird dann zusätzlich zum gespeicherten Korrektursignal beim Abstellen der Brennkraftmaschine der Füllstand des Brennstofftanks erfaßt und gespeichert, und beim Start mit dem Istbefüllungszustand verglichen, wobei noch unterschieden werden kann, wie schnell der Istbefüllungszustand erfaßt wird. Liegt ein Istbefüllungszustand erst nach dem Vorliegen des aktuellen Korrektursignals vor (langsame Tankanzeige), wird folgender Verfahrensablauf A vorgeschlagen, bei einer schnellen Tankanzeige, also bei Vorliegen des Istbefüllungszustandes vor Vorlage des aktuellen Korrektursignals wird ein Verfahrensablauf B vorgegeben.

Verfahrensablauf A (langsame Tankanzeige)

Der Start erfolgt mit Verwendung des letzten gespeicherten Korrektursignals, gleichzeitig wird der beim Abstellen der Brennkraftmaschine gespeicherte Tankbefüllungszustand bewertet, d. h. es wird festgestellt, ob der Brennstofftank, zum Beispiel ≥ halb voll oder < halb voll war.

Bei Feststellung ≥ halb voll wird bis zur Vorlage des aktuellen Korrektursignals das gespeicherte Korrektursignal verwendet, da angenommen wird, daß keine Betankung nach dem Abstellen der Brennkraftmaschine erfolgte, beziehungsweise falls eine Betankung doch erfolgt war, eine Verfälschung durch die zugetankte Menge nicht gravierend erscheint. Bei Feststellung < halb voll wird angenommen, daß eine Betankung erfolgt war und das letzte gespeicherte Korrektursignal wird auf ein mittleres Korrektursignal (zum Beispiel das eines normalen Brennstoffes) nach einer Vorgabefunktion (linear, e-Funktion, treppenförmig u.s.w.) korrigiert.

Nach Vorlage eines aktuellen Korrektursignals der Verdampfungseinrichtung wird sofort mit diesem weitergefahren.

Verfahrensablauf B (schnelle Tankanzeige)

Bei Feststellung der Nichtänderung des Tankbefüllungszustandes wird bis zur Vorlage des aktuellen Korrektursignals das gespeicherte Korrektursignal verwendet.

Bei Feststellung der Änderung des Tankbefüllungszustandes läuft das Verfahren B so weiter wie das Verfahren A im Zustand der Feststellung < halb voll.

Im Betriebsfall einer Lambda = 1-Regelung wird mit stöchiometrischem Gemisch gefahren. In dieser Betriebsphase ist es möglich, das Korrektursignal quantitativ zu überprüfen und im Falle einer Abweichung adaptiv anzugleichen.

**Patentansprüche**

1. Verfahren zur Nutzung von Brennstoffen mit Alkoholzusätzen für eine mit einem elektronischen Gemischbildungssystem betriebene Brennkraftmaschine, bei dem mittels einer Einrichtung zur Verdampfung einer Brennstoffprobenmenge ein Korrektursignal zur Änderung der Gemischzusammensetzung in einem Steuergerät erzeugt wird, dadurch gekennzeichnet, daß das Korrektursignal dadurch ermittelt wird, daß

   - in einem ersten Verfahrensschritt die Brennstoffprobenmenge auf eine vorgegebene Temperatur erhitzt wird, bis die Brennstoffprobenmenge verdampft ist,
   - in einem zweiten Verfahrensschritt die nach der Verdampfung entstandene Volumen- und Temperaturänderung durch Sensoren festgestellt wird und die Sensorwerte
   - in einem dritten Verfahrensschritt mit im Steuergerät gespeicherten Bezugswerten für normalen Brennstoff verglichen wird und aus der Abweichung ein Korrektursignal gebildet wird,
   - in einem vierten Verfahrensschritt mittels des Korrektursignals Motorsteuerungsgrößen änderbar sind und daß
   - in einem fünften Verfahrensschritt das gebildete Korrektursignal gespeichert wird, bis es bei erneuter Brennstoffprobenmessung bestätigt bzw. geändert wird.

2. Verfahren nach dem Oberbegriff des Anspruchs 1, dadurch gekennzeichnet, daß das Korrektursignal dadurch ermittelt wird, daß

   - in einem ersten Verfahrensschritt der Brennstoffprobenmenge ein vorgegebenes Volumen zugeschaltet wird und die Brennstoffprobenmenge auf eine vorgegebene Temperatur erhitzt wird, bis die Brennstoffprobenmenge verdampft ist,
   - in einem zweiten Verfahrensschritt die nach der Verdampfung entstandene Druck- und Temperaturänderung durch Sensoren festgestellt wird und jeder Sensorwert
   - in einem dritten Verfahrensschritt mit im Steuergerät gespeicherten Bezugswerten für normalen Brennstoff verglichen wird und aus der Abweichung ein Korrektursignal gebildet wird,
   - in einem vierten Verfahrensschritt mittels des Korrektursignals Motorsteuerungsgrößen änderbar sind und daß
   - in einem fünften Verfahrensschritt das gebildete Korrektursignal gespeichert wird, bis es bei erneuter Brennstoffprobenmessung bestätigt bzw. geändert wird.

3. Verfahren nach dem Oberbegriff des Anspruchs 1, dadurch gekennzeichnet, daß das Korrektursignal dadurch ermittelt wird, daß

- in einem ersten Verfahrensschritt der Brennstoffprobenmenge ein vorgegebenes Volumen zugeschaltet wird und dieses solange vergrößert wird, bis sich ein vorgegebener Druck in dem Volumen eingestellt hat und die Brennstoffprobenmenge auf eine vorgegebene Temperatur erhitzt wird, bis die Brennstoffprobenmenge verdampft ist,

- in einem zweiten Verfahrensschritt die nach der Verdampfung entstandene Volumen- und Temperaturänderung durch Sensoren festgestellt wird und jeder Sensorwert

- in einem dritten Verfahrensschritt mit im Steuergerät gespeicherten Bezugswerten für normalen Brennstoff verglichen wird und aus der Abweichung ein Korrektursignal gebildet wird,

- in einem vierten Verfahrensschritt mittels des Korrektursignals Motorsteuerungsgrößen änderbar sind und daß

- in einem fünften Verfahrensschritt das gebildete Korrektursignal gespeichert wird, bis es bei erneuter Brennstoffprobenmessung bestätigt bzw. geändert wird.

4. Verfahren nach dem Oberbegriff des Anspruchs 1, dadurch gekennzeichnet, daß das Korrektursignal dadurch ermittelt wird, daß

- in einem ersten Verfahrensschritt der Brennstoffprobenmenge ein vorgegebenes Volumen zugeschaltet wird und die Brennstoffprobenmenge auf eine Temperatur, bei der ein vorgegebenes Volumen erreicht wird, erhitzt wird und hiervon ausgehend die Temperatur erhöht wird, bis die Brennstoffprobenmenge verdampft ist,

- in einem zweiten Verfahrensschritt die nach der Erhitzung bzw. Verdampfung erreichte Temperatur durch einen Sensor festgestellt wird und der Sensorwert

- in einem dritten Verfahrensschritt mit im Steuergerät gespeicherten Bezugswerten für normalen Brennstoff verglichen wird und aus der Abweichung ein Korrektursignal gebildet wird,

- in einem vierten Verfahrensschritt mittels des Korrektursignals Motorsteuerungsgrößen änderbar sind und daß

- in einem fünften Verfahrensschritt das gebildete Korrektursignal gespeichert wird, bis es bei erneuter Brennstoffprobenmessung bestätigt bzw. geändert wird.

5. Verfahren nach einem der vorstehenden Ansprüche 1-4, dadurch gekennzeichnet, daß in einem sechsten Verfahrensschritt die Brennstoffprobenbeheizung abgeschaltet, das Volumen verkleinert und die Einrichtung zur Verdampfung einer Probenmenge gespült wird.

6. Verfahren nach Anspruch 1 bis 5, dadurch gekennzeichnet, daß das im fünften Verfahrensschritt gespeicherte Korrektursignal auch bei einem Wiederstart der Brennkraftmaschine verwendet wird, bis es nach erneuter Brennstoffprobenmessung bestätigt beziehungsweise aktualisiert wird.

7. Verfahren nach Anspruch 1 bis 6, dadurch gekennzeichnet, daß bei einem Start der Brennkraftmaschine ab einem bestimmten Füllstand des Brennstofftanks beim Abstellen der Brennkraftmaschine bis zur Vorlage des aktuellen Korrektursignals kein Korrektursignal oder ein Korrektursignal, das dem des normalen Brennstoffes entspricht, vorgegeben wird.

8. Verfahren nach Anspruch 6 oder 7, dadurch gekennzeichnet, daß bei Vorliegen eines Lambda-Signals eine Adaption des Korrektursignals erfolgen kann.

9. Einrichtung zur Verdampfung einer Brennstoffprobenmenge zur Bildung eines Korrektursignals nach den Verfahren nach Anspruch 1 bis 8 bestehend aus einer beheizbaren, Sensoren aufweisenden Probennahmekammer mit Zu- und Abflußkanälen, dadurch gekennzeichnet, daß die Probennahmekammer (3) sich zu einer Verdampfungskammer (4) aufweitet, in der ein Kolben (10) angeordnet ist, der gegen die Kraft einer Feder (11) axial verschiebbar ist.

10. Einrichtung nach Anspruch 9, dadurch gekennzeichnet, daß die Zu- und Abflußkanäle (5, 6) mit Hilfe von Ventilen (7, 8) geöffnet und geschlossen werden.

11. Einrichtung nach Anspruch 9 oder 10, dadurch gekennzeichnet, daß in der Verdampfungskammer (4) ein Sensor (12) zur Erfassung der Kolbenposition angeordnet ist.

**12.** Einrichtung nach Anspruch 9 oder 10, dadurch gekennzeichnet, daß der Kolben (10) mit Hilfe eines Elektromagneten (14) oder unteratmosphärischen Druck verstellbar ist.

**13.** Einrichtung nach einem der vorstehenden Ansprüche 9, 10 oder 12, dadurch gekennzeichnet, daß in der Verdampfungskammer (4) ein Hubanker (18) angeordnet ist, der über eine Zugfeder (17) mit dem Kolben (10) zusammenwirkt, wobei die Kolbenposition von einem Sensor (12) erfaßt wird.

**14.** Einrichtung nach Anspruch 11 oder 13, dadurch gekennzeichnet, daß der Sensor (12) als Positionsgeber oder Endlagenschalter ausgebildet ist.

# Fig. 1

# Fig. 2

# Fig. 3

**Europäisches
Patentamt**

**EUROPÄISCHER
RECHERCHENBERICHT**

Nummer der Anmeldung

**EP 91 11 5144**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | DE-C-2 259 323   (MOTOREN-WERKE MANNHEIM) * Spalte 3, Zeile 35 - Spalte 4, Zeile 48; Abbildungen 1,2 * * – – – | 1-4,9 | F 02 D 19/08 G 01 N 33/28 |
| A | DE-A-1 523 477   (DAIMLER-BENZ) – – – | | |
| A | GB-A-2 136 118   (INSTITUT FRANCAIS DU PETROLE) – – – – – | | |

RECHERCHIERTE
SACHGEBIETE (Int. Cl.5)

F 02 D
G 01 N

Der vorliegende Recherchenbericht wurde für alle Patentansprüche erstellt

| Recherchenort | Abschlußdatum der Recherche | Prüfer |
|---|---|---|
| Den Haag | 17 Januar 92 | VAN ZOEST A.P. |